# EUROPEAN PATENT APPLICATION

(11) **EP 4 470 540 A1**
(43) Date of publication of application: **04.12.2024**
(21) Application number: 23746197.5
(22) Date of filing: 18.01.2023
(51) Int. Cl.: A61K 31/501, A61K 31/352, A61P 11/00, A61P 11/14

(54) **USE OF HETEROCYCLIC COMPOUND**

(30) Priority: 27.01.2022 CN 202210103132
(71) Applicant: Wuhan Createrna Science and Technology Co.,Ltd., Wuhan, Hubei 430075 (CN)
(72) Inventor: CHEN, Yongkai, Wuhan, Hubei 430075 (CN); GUO, Xiaodan, Wuhan, Hubei 430075 (CN); LOU, Jun, Wuhan, Hubei 430075 (CN); WANG, Chaodong, Wuhan, Hubei 430075 (CN)
(74) Representative: Murgitroyd & Company
(86) International application number: PCT/CN2023/072945
(87) International publication number: WO 2023/143316

(57) **Abstract**

Disclosed is a use of a heterocyclic compound. Specifically disclosed is a use of a heterocyclic compound in the preparation of a drug for treating and/or preventing respiratory diseases; the heterocyclic compound is a substance X, a tautomer thereof, a pharmaceutically acceptable salt thereof, a solvate thereof, or a solvate of the pharmaceutically acceptable salt thereof; the substance X is a compound as shown in formula I or a compound as shown in formula II; the heterocyclic compound of the present invention has a good cough relieving effect.

## Description

The present application claims the right of the priority of Chinese patent application 2022101031321 filed on January 27, 2022. The contents of the above Chinese patent application are incorporated herein by reference in their entireties.

### TECHNICAL FIELD

The present disclosure relates to a use of a heterocyclic compound, and specifically relates to a use of the heterocyclic compound in respiratory diseases, especially cough.

### BACKGROUND

Cough is a common symptom of respiratory system diseases and aids in clearing secretions and harmful factors in the respiratory tract. Patients with chronic cough comprise more than one-third of those affected. The etiology of cough is complex and involves a broad range of factors, particularly in cases of chronic cough where chest imaging examination shows no significant abnormalities. Due to the unclear diagnosis, many patients often undergo various examinations repeatedly or long-term use of large quantities of antibiotics and antitussive medications, with limited efficacy and numerous adverse effects. This significantly impacts patients' work, study, and quality of life, while also imposing a severe burden.

### CONTENT OF THE PRESENT INVENTION

The present disclosure aims to address the issue of the single structure of existing drugs for treating and/or preventing respiratory diseases. To this end, the present disclosure provides a use of a heterocyclic compound in the manufacture of a medicament for treating and/or preventing respiratory diseases. The heterocyclic compound of the present disclosure exhibits a good antitussive effect.

The present disclosure solves the aforementioned technical problem through the following technical solutions.

The present disclosure provides a use of a heterocyclic compound in the manufacture of a medicament for treating and/or preventing respiratory diseases; the heterocyclic compound is a substance X, a tautomer thereof, a pharmaceutically acceptable salt thereof, a solvate thereof, or a solvate of the pharmaceutically acceptable salt thereof; the substance X is a compound of formula I or formula II: 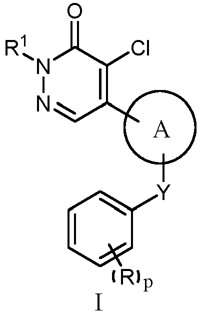
wherein ring A is a 9- to 10-membered heteroaromatic ring containing 3 nitrogen atoms;
Y is -O- or -C(=O)-;
R¹ is H or 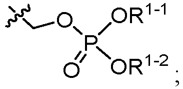
R¹⁻¹ and R¹⁻² are independently H or C₁-C₆ alkyl;
R is independently H, F, -Cl, -Br, -CN, -OR^{a}, -NR^{b}R^{c}, C₁-C₃ alkyl, C₃₋₄ cycloalkyl, C₁-C₃ alkyl substituted by 1, 2, 3, or 4 R²⁻¹, or C₃₋₄ cycloalkyl substituted by 1, 2, 3, or 4 R²⁻²;
R^{a} is independently H or C₁-C₃ alkyl;
R^{b} and R^{c} are independently H or C₁-C₃ alkyl;
R²⁻¹ and R²⁻² are independently F, -Cl, -Br, or -OH;
p is 0, 1, 2, 3, 4, or 5; 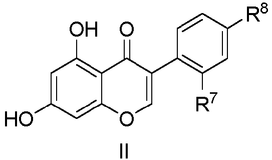
wherein R⁷ is H, F, -Br, or -Cl;
R⁸ is H, F, -Br, -Cl, or -OH.

In a certain embodiment, some groups are defined as follows, and unmentioned groups in the following are as defined in any one of the embodiments according to the present disclosure (hereinafter referred to as "in a certain embodiment"): the compound of formula I has a structure as shown in formula I-A or I-B: 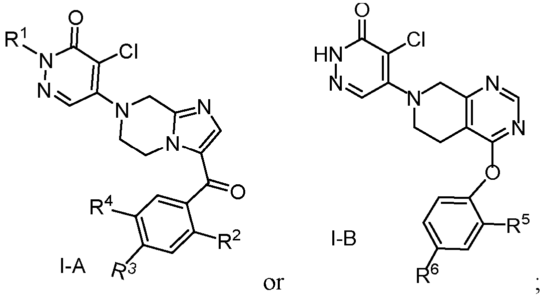
R¹ is as defined in the present disclosure;
R², R³, R⁴, R⁵, and R⁶ are independently H, F, -Cl, -Br, -CN, -OR^{a}, -NR^{b}R^{c}, C₁-C₃ alkyl, C₃₋₄ cycloalkyl, C₁-C₃ alkyl substituted by 1, 2, 3, or 4 R²⁻¹, or C₃₋₄ cycloalkyl substituted by 1, 2, 3, or 4 R²⁻².

In a certain embodiment, R², R³, R⁴, R⁵, and R⁶ are independently H, F, -Cl, -Br, -CN, -OR^{a}, -NR^{b}R^{c}, C₁-C₃ alkyl, C₃₋₄ cycloalkyl, or C₁-C₃ alkyl substituted by 1, 2, 3, or 4 R²⁻¹.

In a certain embodiment, R^{b} and R^{c} are independently C₁-C₃ alkyl.

In a certain embodiment, R²⁻¹ and R²⁻² are F.

In a certain embodiment, p is 1, 2, or 3.

In a certain embodiment, in R¹⁻¹ and R¹⁻², the C₁-C₆ alkyl is independently methyl, ethyl, n-propyl, isopropyl, n-butyl, isobutyl, or tert-butyl, such as tert-butyl.

In a certain embodiment, in R², R³, and R⁴, the C₁-C₃ alkyl is independently methyl, ethyl, n-propyl, or isopropyl, such as methyl.

In a certain embodiment, in R², R³, and R⁴, the C₁-C₃ alkyl substituted by 1, 2, 3, or 4 R²⁻¹ is independently -CH₂F, -CHF₂, -CF₃, -CH₂CH₂F, -CH₂CHF₂, or -CH₂CF₃, such as -CHF₂ or -CF₃.

In a certain embodiment, in R², R³, and R⁴, the C₃₋₄ cycloalkyl is independently cyclopropyl or cyclobutyl, such as cyclopropyl.

In a certain embodiment, in R², R³, and R⁴, the C₃₋₄ cycloalkyl in the "C₃₋₄ cycloalkyl substituted by 1, 2, 3, or 4 R²⁻²" is independently cyclopropyl or cyclobutyl, such as cyclopropyl.

In a certain embodiment, in R^{a}, the C₁-C₃ alkyl is independently methyl, ethyl, n-propyl, or isopropyl, such as methyl.

In a certain embodiment, in R^{b} and R^{c}, the C₁-C₃ alkyl is independently methyl, ethyl, n-propyl, or isopropyl, such as methyl.

In a certain embodiment, R¹⁻¹ and R¹⁻² are independently H, methyl, ethyl, n-propyl, isopropyl, n-butyl, isobutyl, or tert-butyl, such as H or tert-butyl.

In a certain embodiment, R^{a} is independently H, -CH₃, or -CH₂CH₃, such as H or - CH₃.

In a certain embodiment, R^{b} and R^{c} are independently H, -CH₃, or -CH₂CH₃, preferably -CH₃.

In a certain embodiment, R¹ is H,

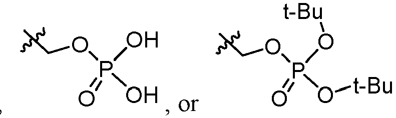

In a certain embodiment, R², R³, and R⁴ are independently H, F, -Cl, -Br, -CN, -OH, - OCH₃, -NH₂, -N(CH₃)₂, -CH₃, cyclopropyl, -CH₂F, -CHF₂, or -CF₃, preferably H, F, -Cl, -CN, -OH, -OCH₃, -N(CH₃)₂, -CH₃, cyclopropyl, -CHF₂, or -CF₃.

In a certain embodiment, R² is H, F, -Cl, -CH₃, -CH₂F, -CHF₂, or -CF₃, preferably F, - CHF₂, or -CF₃.

In a certain embodiment, R³ is H, F, -Cl, -CN, -OH, -OCH₃, -N(CH₃)₂, -CH₃, or cyclopropyl.

In a certain embodiment, R⁴ is H, F, or -CF₃.

In a certain embodiment, R⁵ is F, -Cl, -CH₃, -CHF₂, or -CF₃, preferably -CF₃.

In a certain embodiment, R⁶ is H, F, or -Cl, preferably F.

In a certain embodiment, R⁷ is H.

In a certain embodiment, R⁸ is -OH.

In a certain embodiment,

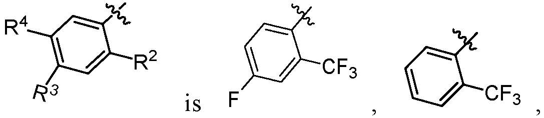

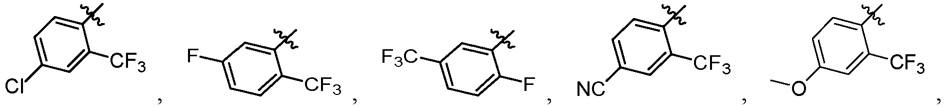

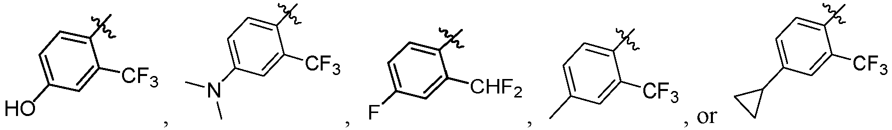

In a certain embodiment, the compound of formula I or formula II is any one of the following compounds:

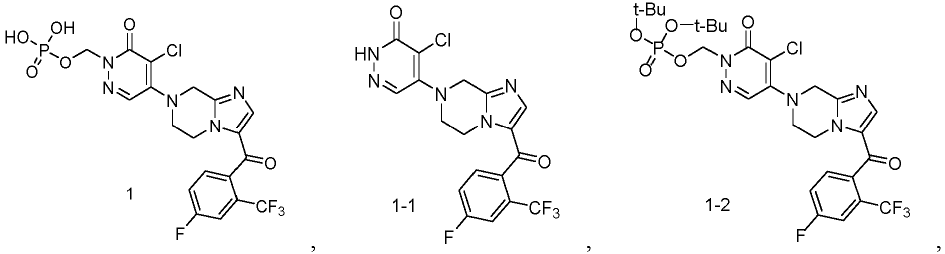

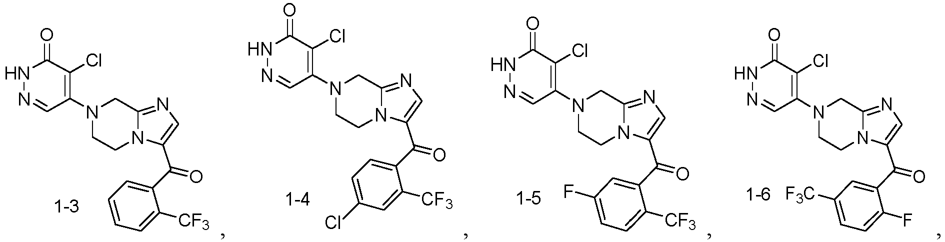

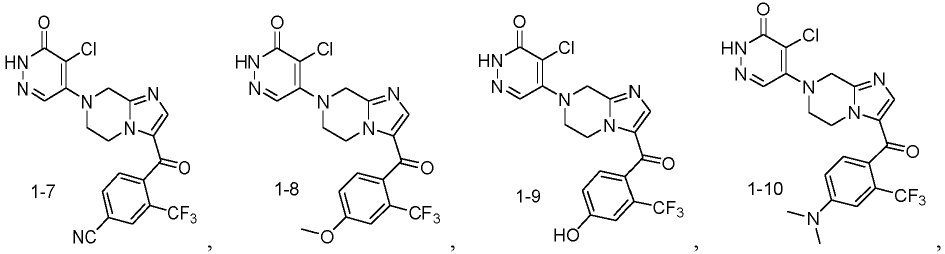

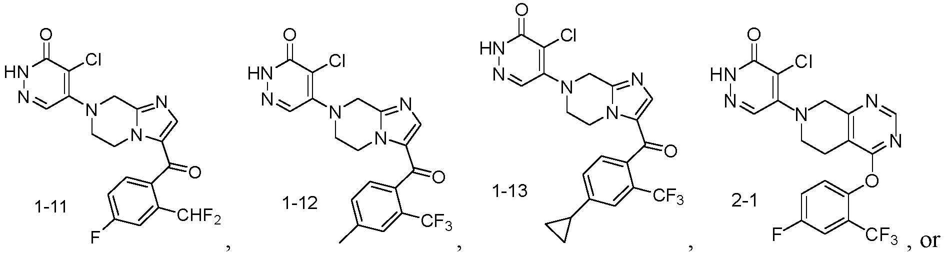

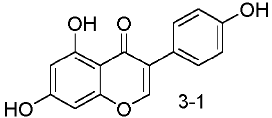

In a certain embodiment, the respiratory disease is a cough, and the cough may be an acute cough, a chronic cough, a refractory chronic cough, or an idiopathic chronic cough.

The present disclosure further provides a method for treating and/or preventing respiratory diseases, which comprises administering a therapeutically effective amount of a heterocyclic compound to a patient, and the heterocyclic compound is as defined above.

In a certain embodiment, the respiratory disease is a cough, and the cough may be an acute cough, a chronic cough, a refractory chronic cough, or an idiopathic chronic cough.

Unless otherwise stated, the terms used in the description and claims of the present disclosure have the following meanings:
The term "pharmaceutically acceptable salt" refers to the salt prepared by the compound of the present disclosure and a relatively nontoxic and pharmaceutically acceptable acid or base. When the compound of the present disclosure contains a relatively acidic functional group, a base addition salt can be obtained by bringing the prototype form of the compound into contact with a sufficient amount of the pharmaceutically acceptable base in a pure solution or a suitable inert solvent. Pharmaceutically acceptable base addition salts include, but are not limited to, lithium salts, sodium salts, potassium salts, calcium salts, aluminum salts, magnesium salts, zinc salts, bismuth salts, ammonium salts, diethanolamine salts. When the compound of the present disclosure contains a relatively basic functional group, an acid addition salt can be obtained by bringing the prototype form of the compound into contact with a sufficient amount of pharmaceutically acceptable acid in a pure solution or a suitable inert solvent. The pharmaceutically acceptable acids include inorganic acids, including but not limited to: hydrochloric acid, hydrobromic acid, hydroiodic acid, nitric acid, carbonic acid, phosphoric acid, phosphorous acid, sulfuric acid, etc. The pharmaceutically acceptable acids include organic acids, and the organic acids include, but are not limited to: acetic acid, propionic acid, oxalic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, salicylic acid, tartaric acid, methanesulfonic acid, isonicotinic acid, oleic acid, tannic acid, pantothenic acid, hydrogen tartrate, ascorbic acid, gentisic acid, fumaric acid, gluconic acid, sugar acid, formic acid, ethanesulfonic acid, pamoic acid (i.e., 4,4'-methylene-bis(3-hydroxy-2-naphthoic acid)), amino acids (such as glutamic acid, arginine), etc. When the compounds of the present disclosure contain relatively acidic and relatively basic functional groups, they can be converted into base addition salts or acid addition salts. For details, see Berge et al., "Pharmaceutical Salts", Journal of Pharmaceutical Science 66: 1-19 (1977), or Handbook of Pharmaceutical Salts: Properties, Selection, and Use (P. Heinrich Stahl and Camille G. Wermuth, ed., Wiley-VCH, 2002).

The term "solvate" refers to a substance formed by combining a compound of the present disclosure with a stoichiometric or non-stoichiometric solvent. Solvent molecules in the solvate can exist in an ordered or unordered arrangement. The solvent includes, but is not limited to, water, methanol, ethanol, etc.

The "pharmaceutically acceptable salt" and "solvate" in the term "solvate of pharmaceutically acceptable salt" are defined as above, and the solvate of pharmaceutically acceptable salt refers to a substance formed by combining the compound of the present disclosure: 1, with a relatively nontoxic and pharmaceutically acceptable acid or base, and, 2, with a stoichiometric or non-stoichiometric solvent. The "solvate of pharmaceutically acceptable salt" includes, but is not limited to, the hydrochloride monohydrate of the compound of the present disclosure.

The term "alkyl" refers to a saturated linear or branched monovalent hydrocarbon group, where the alkyl includes C₁-C₆ alkyl, C₁-C₄ alkyl, C₁-C₃ alkyl, etc. Examples of the alkyl include, but are not limited to, methyl, ethyl, n-propyl, isopropyl, n-butyl, isobutyl, or tert-butyl.

The term "cycloalkyl" refers to a saturated cyclic hydrocarbon group, where the cycloalkyl includes C₃₋₆ cycloalkyl, C₃₋₅ cycloalkyl, C₃₋₄ cycloalkyl, etc. Examples of the cycloalkyl include, but are not limited to, cyclopropyl or cyclobutyl.

The term "heteroaromatic ring" refers to a conjugated ring system group containing carbon atoms and heteroatoms selected from nitrogen, oxygen, and sulfur. At least one ring possesses aromaticity; examples include groups similar to

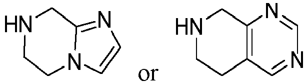

The term "treatment" refers to therapeutic therapy. When referring to a specific disorder, treatment refers to: (1) ameliorating one or more biological manifestations of the disease or disorder, (2) interfering with (a) one or more points in the biological cascade leading to or causing the disorder or (b) one or more biological manifestations of the disorder, (3) ameliorating one or more symptoms, effects, or side effects associated with the disorder, or one or more symptoms, effects or side effects associated with the disorder or its treatment, or (4) slowing the progression of the disorder or one or more biological manifestations of the disorder.

The term "prevention" refers to the reduction of the risk of acquiring or developing diseases or disorders.

The term "patient" refers to any animal that will or has received the administration of the compound or composition according to the example of the present disclosure, preferably a mammal. The term "mammal" includes any mammal. Examples of mammals include, but are not limited to, cows, horses, sheep, pigs, cats, dogs, mice, rats, rabbits, guinea pigs, monkeys, humans, etc., preferably humans.

The term "therapeutically effective amount" refers to the amount of a compound that is sufficient to effectively treat the diseases or disorders described herein when administered to a patient. The "therapeutically effective amount" will vary according to the compound, the disease and its severity, and the age of the patient to be treated, but it can be adjusted by those skilled in the art as needed.

On the basis of not violating the common sense in the field, the preferred conditions above can be arbitrarily combined to obtain the preferred examples of the present disclosure.

The reagents and raw materials used in the present disclosure are commercially available.

The positive and progressive effect of the present disclosure lies in that the heterocyclic compound of the present disclosure exhibits a good antitussive effect.

### DETAILED DESCRIPTION OF THE PREFERRED EMBODIMENT

### Example 1: Preparation of compound (5-chloro-4-(3-(4-fluoro-2-(trifluoromethyl)benzoyl)-5,6-dihydroimidazo[1,2-a]pyrazin-7(8H)-yl)-6-oxopyridazin-1(6H)-yl)methyl dihydrogen phosphate (compound 1)

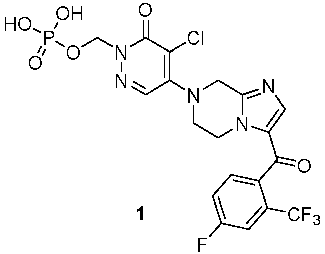

### Synthesis route:

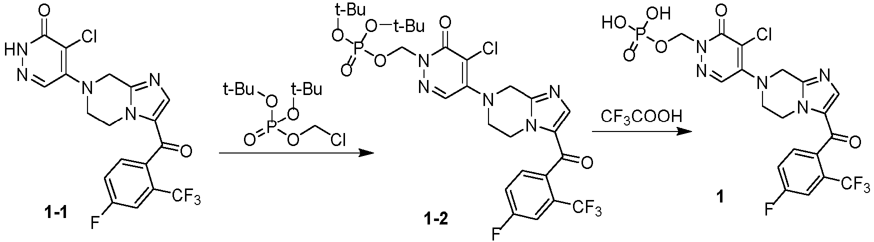

### 1.1 Preparation of compound 1-2

To a reaction flask were added compound **1-1** (300 mg, 0.68 mmol), sodium iodide (132 mg, 0.88 mmol), and tetrahydrofuran (3 mL), and the mixture was stirred until the reactant was dissolved, and then cooled to about 0°C under an ice bath. To the reaction mixture was added lithium tert-butoxide (1 M in tetrahydrofuran, 0.82 mL, 0.82 mmol) dropwise, and the dropwise addition was completed. Then di-tert-butyl chloromethylphosphonate (230 mg, 0.83 mmol) was added to the reaction mixture, and the reaction mixture was warmed to room temperature and reacted for 12 hours. The reaction mixture was quenched with water, extracted with ethyl acetate (5 mL * 3), and the organic phase was washed with saturated brine (10 mL), dried over anhydrous sodium sulfate, filtered, then the filtrate was concentrated under reduced pressure to obtain compound **1-2** (300 mg), which was directly used in the next reaction step without further purification.

LC-MS [M+H]⁺ = 664.2.

### 1.2 Preparation of compound (5-chloro-4-(3-(4-fluoro-2-(trifluoromethyl)benzoyl)-5,6-dihydroimidazo[1,2-a]pyrazin-7(8H)-yl)-6-oxopyridazin-1(6H)-yl)methyl dihydrogen phosphate (compound 1)

Compound **1-2** (300 mg, 0.45 mmol) was dissolved in dichloromethane (3 mL). To the reaction mixture was added trifluoroacetic acid (0.6 mL) dropwise with stirring, then the mixture was stirred at 25°C for 1 hour. After the reaction was completed, the mixture was concentrated under reduced pressure to obtain a crude product, and the crude product was purified by preparative high performance liquid chromatography to obtain compound **1** (112.3 mg).

LC-MS [M+H]⁺ = 552.0; ¹H NMR (400 MHz, DMSO-*d₆*): δ 8.14 (s, 1H), 7.84 (dd, *J* = 9.2, 2.8 Hz, 1H), 7.79 (dd, *J =* 8.4, 5.6 Hz, 1H), 7.68 (td, *J =* 8.4, 2.6 Hz, 1H), 7.36 (s, 1H), 5.68 (d, *J =* 7.6 Hz, 2H), 4.91 (s, 2H), 4.54 (t, *J =* 5.6 Hz, 2H), 3.98 (t, *J =* 5.6 Hz, 2H).

### Effect example:

The structures of compounds 2-1 and 3-1 used in the following effect examples are as follows:

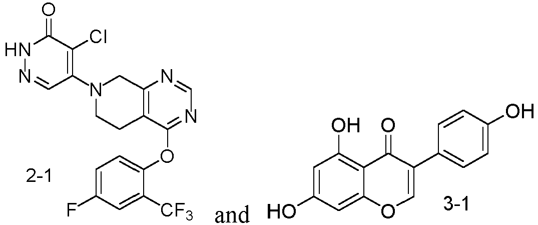

### Effect example 1: Pharmacodynamic study of citric acid-induced acute cough in guinea pigs

### (1) Experimental samples are as follows:

Male Hartley guinea pigs: Beijing Vital River Laboratory Animal Technology Co., Ltd;
250 µM ATP solution: Sigma, catalog number A2383;
0.5 M citric acid solution: Sigma, catalog number C2404;
DSI Buxco Whole Body Plethysmography (WBP) system instrument: manufacturer DSI, serial numbers 100249, 990171;
Electronic balance: Mettler Toledo, serial number B844687071.

### (2) Preparation of test substance:

Solvent (10% (v/v) E-TPGS aqueous solution, pH 7.4 ± 0.1): TPGS was heated and melted into a liquid in a water bath. 10 mL of TPGS was taken and then added to 85 mL of deionized water, and the mixture was stirred to form a homogeneous solution. The pH was adjusted to 7.4 ± 0.1 using an appropriate concentration of HCl/NaOH. The final volume was made up to 100 mL with deionized water, and the mixture was stirred until visually homogeneous. The pH was checked, and if necessary, adjusted to 7.4 ± 0.1 using an appropriate concentration of HCl/NaOH.

The administration formulation was prepared under yellow light conditions.

The required mass of the compound was accurately weighed into an appropriate container, and slowly added to a suitable container containing 80% of the final volume of the solvent (10% (v/v) E-TPGS aqueous solution, pH 7.4 ± 0.1), and the mixture was stirred until visually homogeneous. The solvent was added to 90% of the final volume, and the mixture was stirred until visually homogeneous. If necessary, the pH was adjusted to 7.3-7.6 with an appropriate concentration of HCl/NaOH. If necessary, the formulation was subjected to ultrasonic treatment. The solvent was then added to the final volume, and the mixture was stirred until a visually clear and homogeneous solution was obtained.

After the preparation of the administration formulation was completed, the administration formulation was aliquoted into daily doses and stored in a refrigerator at 2-8°C, protected from light, and used within 10 days. Prior to administration, the formulation was removed from the refrigerator 30 minutes in advance, and stirred for 30 minutes at room temperature and protected from light until it reached room temperature.

Preparation of dextromethorphan: the required volume for administration was calculated based on the animal's body weight. The weighting amount of dextromethorphan (DM) was determined based on the administration volume and dose. The DM was accurately weighed and dissolved in 1×PBS (Phosphate Buffered Saline) with the calculated administration volume. The solution was mixed homogeneously by shaking until a clear solution was formed, and it was prepared freshly before use.

### (3) Experimental procedure

In the experiment, the solvent (10% (v/v) E-TPGS (Vitamin E Polyethylene Glycol Succinate)) and each test compound were administered to guinea pigs by gavage 3 hours prior to citric acid nebulization. The reference compound, dextromethorphan (DM), was administered to guinea pigs by gavage 40 minutes prior to citric acid nebulization (see Table 1 for details).

At the scheduled time, male Hartley guinea pigs (300-400 g) were placed in the respiratory volume plethysmography chamber of the DSI Buxco Whole Body Plethysmography (WBP) system. After an acclimation period of 3-5 minutes, a solution of ATP with a concentration of 250 µM was nebulized and sprayed into the chamber for 2 minutes. After a 3-minute interval, a solution of citric acid with a concentration of 0.5 M was administered for 5 minutes to induce coughing. From the start of citric acid nebulization, the WBP system recorded the total cough counts (CCnt) within 10 minutes and the cough incubation period (CIP, i.e., the time from the start of citric acid induction to the appearance of the first cough).

**Table 1. Grouping and dosing regimen for pharmacodynamic testing in the ATP-sensitized citric acid-induced cough model in guinea pigs**

| **Group** | **Group** | **Number of animals (n)** | **Modeling method (Nebulization/ Single dose)** | **Administration method/ Administration frequency** | **Administration volume (mL/kg)** |
|---|---|---|---|---|---|
| **1** | Solvent (10% (v/v) E-TPGS) | 12 | 250 µM ATP solution + 0.5 M citric acid solution | 3 hours before citric acid nebulization Oral; single dose | 5 |
| **2** | DM: 63 mg/kg | 12 | 250 µM ATP solution + 0.5 M citric acid solution | 40 minutes before citric acid nebulization Oral; single dose | 5 |
| **3** | Compound 1: 3 mg/kg | 12 | 250 µM ATP solution + 0.5 M citric acid solution | 3 hours before citric acid nebulization Oral; single dose | 5 |
| **4** | Compound 1: 10 mg/kg | 12 | 250 µM ATP solution + 0.5 M citric acid solution | 3 hours before citric acid nebulization Oral; single dose | 5 |
| **5** | Compound 1: 30 mg/kg | 12 | 250 µM ATP solution + 0.5 M citric acid solution | 3 hours before citric acid nebulization Oral; single dose | 5 |
| **6** | Compound 2-1: 30 mg/kg | 12 | 250 µM ATP solution + 0.5 M citric acid solution | 3 hours before citric acid nebulization Oral; single dose | 5 |
| **7** | Compound 3-1: 30 mg/kg | 12 | 250 µM ATP solution + 0.5 M citric acid solution | 3 hours before citric acid nebulization Oral; single dose | 5 |

As shown in Tables 2 and 3, after the intervention with the reference compound DM, ATP solution and citric acid solution were given to guinea pigs to induce coughing by nebulization. The average cough times of the guinea pigs was 12.17, and the average CIP of the guinea pigs was 291.00 seconds. This was significantly less than the average cough times in the solvent group, which was 21.50 times (p = 0.0076), and longer than the average CIP of the solvent group, which was 190.75 s, indicating the successful establishment of the model.

Upon administration of compound 1 at doses of 3 mg/kg, 10 mg/kg, and 30 mg/kg, the average cough times of guinea pigs were 17.00, 13.42, and 10.73, respectively, decreased by 20.93%, 37.60%, and 50.11% compared to the solvent group, respectively. Both the 10 mg/kg and 30 mg/kg dose groups showed statistically significant differences (p = 0.0005, p = 0.0001), demonstrating a good antitussive effect. Meanwhile, compared to the solvent group, compound 1 at doses of 3 mg/kg, 10 mg/kg, and 30 mg/kg significantly prolonged the CIP, with CIP of 234.42 s, 283.67 s, and 293.75 s, respectively, indicating a good antitussive efficacy.

Moreover, compounds 2-1 and 3-1 also exhibited good antitussive efficacy. The average cough times decreased by 40.70% (p = 0.0016) and 44.96% (p = 0.0010), respectively, compared to the solvent group. The CIP for compounds 2-1 and 3-1 were 290.08 sand 281.67 s, respectively, showing a significant prolongation compared to the solvent group.

**Table 2. Effect of compounds on total cough count (CCnt) in citric acid-induced acute cough in guinea pigs**

| **Group** | **Total cough count (CCnt)^{a} / times** | **Change rate^{b} (%)** | ***p*-value** |
|---|---|---|---|
| Solvent (10% (v/v) E-TPGS) | 21.50 ± 1.57 | - | - |
| DM: 63 mg/kg | 12.17 ± 2.76^{##**c**} | -43.41 | 0.0076 |
| Compound 1: 3 mg/kg | 17.00 ± 2.11 | -20.93 | 0.1011 |
| Compound 1: 10 mg/kg | 13.42 ± 1.18^{###} | -37.60 | 0.0005 |
| Compound 1: 30 mg/kg | 10.73 ± 1.71^{###} | -50.11 | 0.0001 |
| Compound 2-1: 30 mg/kg | 12.75 ± 1.86^{###} | -40.70 | 0.0016 |
| Compound 3-1: 30 mg/kg | 11.83 ± 2.02^{###} | -44.96 | 0.0010 |

| | | | |
|---|---|---|---|
| Note: a. Mean ± standard error of the mean (Mean ± SEM), N = 12; b. Change Rate (%) = (Average Total Cough Count in Compound Administration Group - Average Total Cough Count in Solvent Group) / Average Total Cough Count in Solvent Group × 100%; c. ## indicates p < 0.01, ### indicates p < 0.005: compared to the solvent group (t-test). | | | |

**Table 3. Effect of compounds on cough incubation period (CIP) of citric acid-induced cough in guinea pigs**

| **Group** | **Cough incubation period (CIP) / seconds** |
|---|---|
| Solvent (10% (v/v) E-TPGS) | 190.86 ± 22.15 |
| DM: 63 mg/kg | 291.00 ± 41.65 |
| Compound 1: 3 mg/kg | 234.42 ± 24.11 |
| Compound 1: 10 mg/kg | 283.67 ± 42.98 |
| Compound 1: 30 mg/kg | 293.75 ± 33.66 |
| Compound 2-1: 30 mg/kg | 290.08 ± 43.61 |
| Compound 3-1: 30 mg/kg | 281.67 ± 37.84 |

In summary, the research results indicate that compared to the solvent group, the compounds of the present disclosure significantly reduced the total cough count (CCnt) induced by citric acid in guinea pigs and prolonged the cough incubation period (CIP), demonstrating good antitussive effects.

## Claims

1. A use of a heterocyclic compound in the manufacture of a medicament for treating and/or preventing respiratory diseases; the heterocyclic compound is a substance X, a tautomer thereof, a pharmaceutically acceptable salt thereof, a solvate thereof, or a solvate of the pharmaceutically acceptable salt thereof; the substance X is a compound of formula I or formula II: 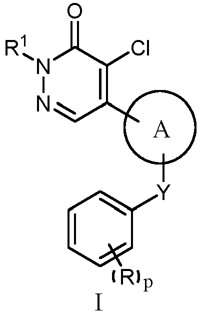
wherein ring A is a 9- to 10-membered heteroaromatic ring containing 3 nitrogen atoms; Y is -O- or -C(=O)-;
R¹ is H or 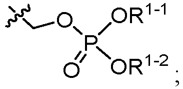
R¹⁻¹ and R¹⁻² are independently H or C₁-C₆ alkyl;
R is independently H, F, -Cl, -Br, -CN, -OR^{a}, -NR^{b}R^{c}, C₁-C₃ alkyl, C₃₋₄ cycloalkyl, C₁-C₃ alkyl substituted by 1, 2, 3, or 4 R²⁻¹, or C₃₋₄ cycloalkyl substituted by 1, 2, 3, or 4 R²⁻²;
R^{a} is independently H or C₁-C₃ alkyl;
R^{b} and R^{c} are independently H or C₁-C₃ alkyl;
R²⁻¹ and R²⁻² are independently F, -Cl, -Br, or -OH;
p is 0, 1, 2, 3, 4, or 5; 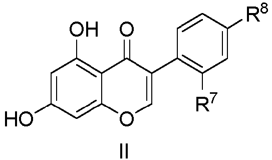
wherein R⁷ is H, F, -Br, or -Cl;
R⁸ is H, F, -Br, -Cl, or -OH.

2. The use of the heterocyclic compound in the manufacture of the medicament for treating and/or preventing respiratory diseases according to claim 1, wherein the compound of formula I has a structure as shown in formula I-A or I-B: 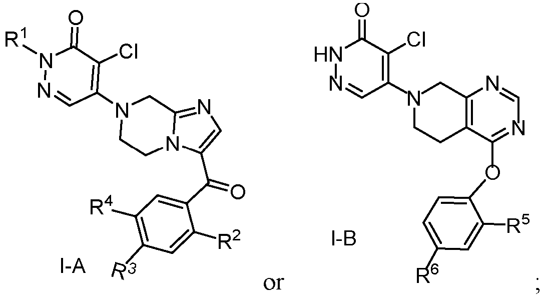
R¹ is H or 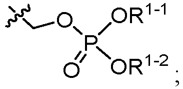
R¹⁻¹ and R¹⁻² are independently H or C₁-C₆ alkyl;
R², R³, R⁴, R⁵, and R⁶ are independently H, F, -Cl, -Br, -CN, -OR^{a}, -NR^{b}R^{c}, C₁-C₃ alkyl, C₃₋₄ cycloalkyl, C₁-C₃ alkyl substituted by 1, 2, 3, or 4 R²⁻¹, or C₃₋₄ cycloalkyl substituted by 1, 2, 3, or 4 R²⁻².

3. The use of the heterocyclic compound in the manufacture of the medicament for treating and/or preventing respiratory diseases according to claim 2, wherein the use satisfies one or more of the following conditions:
(1) R², R³, R⁴, R⁵, and R⁶ are independently H, F, -Cl, -Br, -CN, -OR^{a}, -NR^{b}R^{c}, C₁-C₃ alkyl, C₃₋₄ cycloalkyl, or C₁-C₃ alkyl substituted by 1, 2, 3, or 4 R²⁻¹;
(2) R^{b} and R^{c} are independently C₁-C₃ alkyl;
(3) R²⁻¹ and R²⁻² are F.

4. The use of the heterocyclic compound in the manufacture of the medicament for treating and/or preventing respiratory diseases according to claim 1 or 2, wherein the use satisfies one or more of the following conditions:
(1) in R¹⁻¹ and R¹⁻², the C₁-C₆ alkyl is independently methyl, ethyl, n-propyl, isopropyl, n-butyl, isobutyl, or tert-butyl;
(2) in R², R³, and R⁴, the C₁-C₃ alkyl is independently methyl, ethyl, n-propyl, or isopropyl;
(3) in R², R³, and R⁴, the C₁-C₃ alkyl substituted by 1, 2, 3, or 4 R²⁻¹ is independently - CH₂F, -CHF₂, -CF₃, -CH₂CH₂F, -CH₂CHF₂, or -CH₂CF₃;
(4) in R², R³, and R⁴, the C₃₋₄ cycloalkyl is independently cyclopropyl or cyclobutyl;
(5) in R^{a}, the C₁-C₃ alkyl is independently methyl, ethyl, n-propyl, or isopropyl;
(6) in R^{b} and R^{c}, the C₁-C₃ alkyl is independently methyl, ethyl, n-propyl, or isopropyl.

5. The use of the heterocyclic compound in the manufacture of the medicament for treating and/or preventing respiratory diseases according to claim 4, wherein the use satisfies one or more of the following conditions:
(1) in R¹⁻¹ and R¹⁻², the C₁-C₆ alkyl is tert-butyl;
(2) in R², R³, and R⁴, the C₁-C₃ alkyl is methyl;
(3) in R², R³, and R⁴, the C₁-C₃ alkyl substituted by 1, 2, 3, or 4 R²⁻¹ is independently - CHF₂ or -CF₃;
(4) in R², R³, and R⁴, the C₃₋₄ cycloalkyl is cyclopropyl;
(5) in R^{a}, the C₁-C₃ alkyl is methyl;
(6) in R^{b} and R^{c}, the C₁-C₃ alkyl is methyl.

6. The use of the heterocyclic compound in the manufacture of the medicament for treating and/or preventing respiratory diseases according to claim 1, wherein the use satisfies one or more of the following conditions:
(1) R¹⁻¹ and R¹⁻² are independently H, methyl, ethyl, n-propyl, isopropyl, n-butyl, isobutyl, or tert-butyl;
(2) R^{a} is independently H, -CH₃, or -CH₂CH₃;
(3) R^{b} and R^{c} are independently H, -CH₃, or -CH₂CH₃;
(4) p is 1, 2, or 3.

7. The use of the heterocyclic compound in the manufacture of the medicament for treating and/or preventing respiratory diseases according to claim 6, wherein the use satisfies one or more of the following conditions:
(1) R¹⁻¹ and R¹⁻² are independently H or tert-butyl;
(2) R^{a} is independently H or -CH₃;
(3) R^{b} and R^{c} are -CH₃.

8. The use of the heterocyclic compound in the manufacture of the medicament for treating and/or preventing respiratory diseases according to claim 2, wherein R², R³, and R⁴ are independently H, F, -Cl, -Br, -CN, -OH, -OCH₃, -NH₂, -N(CH₃)₂, -CH₃, cyclopropyl, -CH₂F, - CHF₂, or -CF₃, preferably H, F, -Cl, -CN, -OH, -OCH₃, -N(CH₃)₂, -CH₃, cyclopropyl, -CHF₂, or -CF₃.

9. The use of the heterocyclic compound in the manufacture of the medicament for treating and/or preventing respiratory diseases according to claim 2, wherein the use satisfies one or more of the following conditions:
(1) R¹ is H, 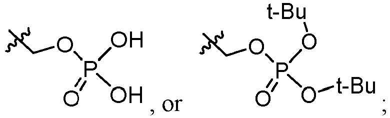
(2) R² is H, F, -Cl, -CH₃, -CH₂F, -CHF₂, or -CF₃, preferably F, -CHF₂, or -CF₃;
(3) R³ is H, F, -Cl, -CN, -OH, -OCH₃, -N(CH₃)₂, -CH₃, or cyclopropyl;
(4) R⁴ is H, F, or -CF₃;
(5) R⁵ is F, -Cl, -CH₃, -CHF₂, or -CF₃, preferably -CF₃;
(6) R⁶ is H, F, or -Cl, preferably F;
(7) R⁷ is H;
(8) R⁸ is -OH.

10. The use of the heterocyclic compound in the manufacture of the medicament for treating and/or preventing respiratory diseases according to claim 2, wherein

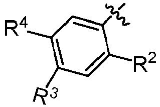

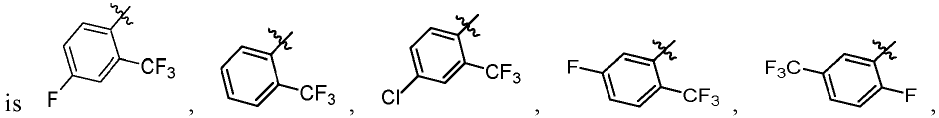

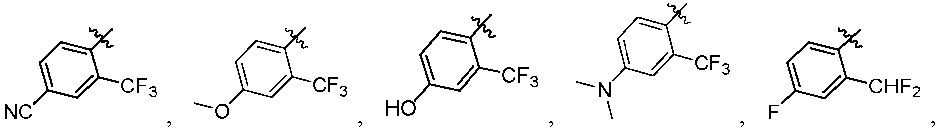

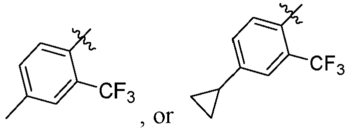

11. The use of the heterocyclic compound in the manufacture of the medicament for treating and/or preventing respiratory diseases according to claim 1, wherein the compound of formula I or formula II is any one of the following compounds: 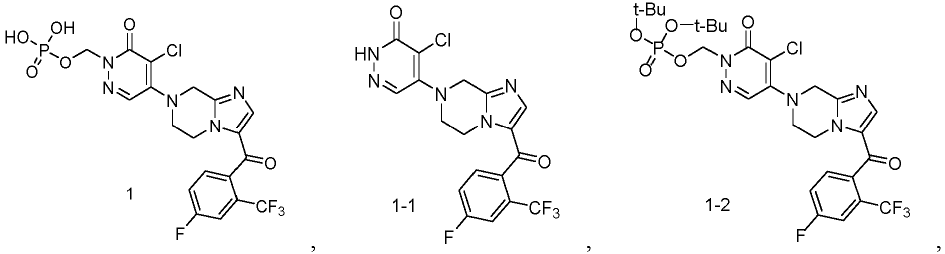 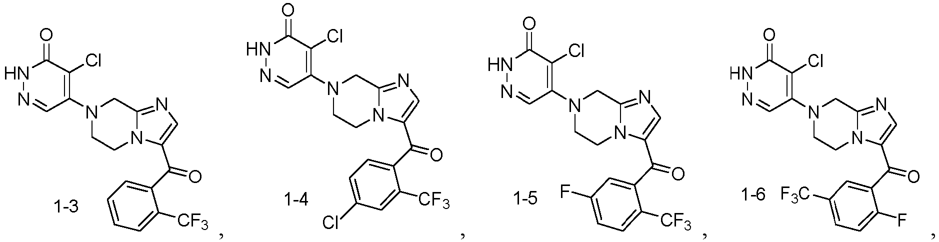 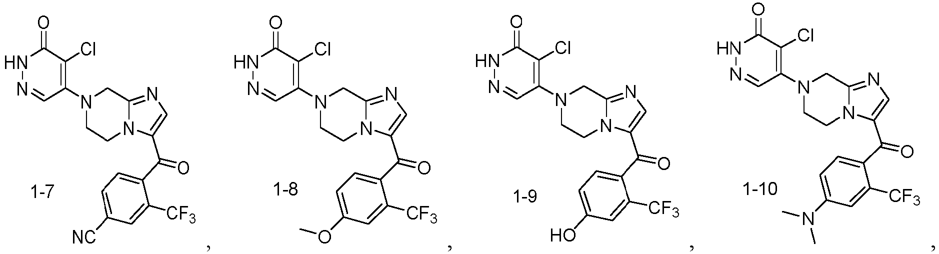 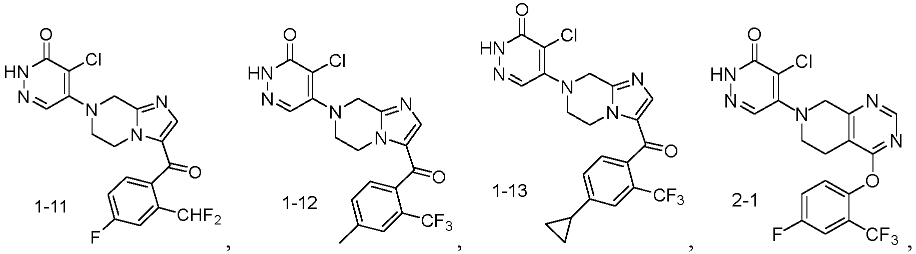 or

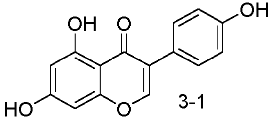

12. The use of the heterocyclic compound in the manufacture of the medicament for treating and/or preventing respiratory diseases according to any one of claims 1 to 11, wherein the respiratory disease is a cough, and the cough is preferably an acute cough, a chronic cough, a refractory chronic cough, or an idiopathic chronic cough.
